# EUROPEAN PATENT SPECIFICATION

(11) **EP 3 727 196 B1**
(45) Date of publication and mention of the grant of the patent: **19.04.2023**
(21) Application number: 18890474.2
(22) Date of filing: 17.12.2018
(51) Int. Cl.: A61F 2/07, A61F 2/04, A61F 2/06

(54) **BRANCH POINT FLOW DIVERSION DEVICE**
FLUSSABZWEIGUNGSVORRICHTUNG FÜR ZWEIGPUNKT
DISPOSITIF DE DÉVIATION D'ÉCOULEMENT DE POINT DE BRANCHEMENT

(30) Priority: 18.12.2017 US 201762607019 P; 24.05.2018 US 201862676168 P
(43) Date of publication of application: 28.10.2020
(73) Proprietor: THRU-FLO ENDOVASCULAR, INC., Santa Monica, CA 90402 (US)
(72) Inventor: TEITELBAUM, George P., Santa Monica, CA 90402 (US)
(74) Representative: Hermann, Felix
(86) International application number: PCT/US2018/066048
(87) International publication number: WO 2019/126060

(56) References cited:
- CA-A1- 2 294 735
- US-A- 5 669 924
- US-A1- 2006 190 070
- US-A1- 2007 270 902
- US-A1- 2008 097 495
- US-A1- 2014 249 620
- US-B1- 6 322 587
- US-B1- 8 690 907
- US-B2- 9 474 640

## Description

### Technical Field

The field of the present disclosure relates generally to medical devices, and in particular, to flow diversion devices that may be used for treatment of wide-neck and fusiform aneurysms.

### Background

Flow diversion devices, such as the Pipeline embolization device, the Surpass flow diverter, and the Silk flow diverter, are stent-like devices composed of tightly braided, thin-wire elements typically used for the treatment of intracranial aneurysms. These devices are employed endovascularly to treat aneurysms by diverting blood flow away from the aneurysm to induce aneurysm thrombosis, which helps prevent rupture of the aneurysm and may eventually result in the gradual shrinkage and occlusion of the aneurysm. In addition, when used for fusiform aneurysms (i.e., aneurysms with no definable neck), the flow diversion device may promote reconstruction of a smooth endothelial covered channel in continuation with the parent artery. While the flow diversion device directs blood away from the aneurysm, the thin-wire braided design allows modest through-flow of blood to maintain the patency of important small arterial side branches adjacent to the treated aneurysm.

Large intracranial aneurysms (which range in diameter from 10-25 mm) and particularly giant intracranial aneurysms (those greater than 25 mm in diameter) frequently have a wide-neck (dome-to-neck diameter ratio of less than 2) or are fusiform. Typically, large and giant aneurysms have poor occlusion, rupture, and survival rates regardless of the form of therapy (e.g., open brain surgery or other endovascular techniques) used to treat them. The use of finely braided stent devices to divert blood flow away from aneurysms has yielded promising treatment results compared to open surgery and other conventional endovascular techniques, such as coil embolization with or without the assistance of an endovascular stent or balloon remodeling.

CA 2294735 A1 discloses an occlusion system for treatment an aneurysm in a parent vessel. The parent vessel defines a lumen that has a lumen wall. The aneurysm has a neck in communication with the lumen. The occlusion system includes a stent configured for deployment in the parent vessel. The stent has at least a first portion and a second portion. The first portion is permeable to blood flow and is arranged such that, when the stent is deployed, the first portion is spaced from the neck of the aneurysm. The second portion is less permeable to blood flow than the first portion and is arranged such that, when the stent is deployed, the second portion overlies the neck of the aneurysm.

US 2006/190070 A1 discloses devices, systems and methods for stenting body lumens. In particular, stents are provided which are advanceable directly over a guidewire and expandable within a target location of a body lumen by retraction of the guidewire and/or by releasing constraining element(s) disposed around at least a portion of the stent. Typically the constraining element(s) have the form of one or more bands or layers of material which hold the stent in an unexpanded configuration. These stent designs allow delivery to a body lumen without the need for a number of additional devices which are typically used in the delivery of conventional stents, thereby reducing the profile of the stent during delivery, increasing the flexibility of the stent during delivery to allow passage through more tortuous pathways, and allowing the delivery of branched or otherwise connected stents to body lumens, such as branched lumens.

### Summary

The invention is defined by the subject matter of independent claim 1. Advantageous embodiments are subject to the dependent claims.

### Brief Description of the Drawings

FIG. 1 illustrates an example flow diversion device according to one embodiment. FIG. 1A is an enlarged view of a portion of the flow diversion device of FIG. 1.
FIG. 2 is a cross-section view of the flow diversion device of FIG. 1.
FIGS. 3, 4, and 5 illustrate an example assembly and delivery of the flow diversion device of FIG. 1 via a catheter and pusher wire according to one embodiment.
FIGS. 6A and 6B illustrate schematic views of the flow diversion device in position at a delivery site for treatment of an aneurysm.
FIGS. 7 and 8 collectively illustrate an example of the flow diversion device, according to another embodiment.
FIG. 9 illustrates an example embodiment of the flow diversion device that may be used for carotid and coronary bypass grafting.
FIG. 10 illustrates an example embodiment of a flow diversion device with a flexible hypotube for delivering embolic particles through the flow diversion device.
FIG. 11 illustrates an example embodiment of a flow diversion device with features for improving flow to larger side branches.
FIGS. 12, 13, and 14 illustrate an example delivery process for the flow diversion device of FIG. 11 to a treatment site.
FIG. 15A illustrates an example embodiment of a collapsed flow diversion device with a plurality of longitudinal slits.
FIG. 15B illustrates a detailed view of proximal end of the collapsed flow diversion device of FIG. 15A.
FIG. 15C illustrates a detailed view of a distal end of the collapsed flow diversion device of FIG. 15A.
FIG. 16 illustrates the flow diversion device of 15A in an expanded configuration.
FIG. 17A illustrates a slit with an opposing Y-shaped slit in an collapsed configuration and an expanded hourglass shaped slit in an expanded configuration.
FIG. 17B illustrates a slit with a X-shape slit in an collapsed configuration and an hourglass shaped slit in an expanded configuration.
FIG. 17C illustrates a slit with a Y-shaped slit in an collapsed configuration and a V-shaped slit in an expanded configuration.
FIG. 18A illustrates a branch point aneurysm.
FIG. 18B illustrates an exemplary fenestrated flow diversion device with a central bulge created by a centrally bulging stent-scaffold.
FIG. 19 illustrates an exemplary fenestrated flow diversion device with a central bulge created by a centrally bulging stent-scaffold for treating a branch point aneurysm.
FIG. 20A illustrates an Y-construct flow diversion device according to one embodiment of the invention.
FIG. 20B illustrates a Y-construct flow diversion device according to one embodiment of the invention.
FIG. 20C illustrates a Y-construct flow diversion device according to one embodiment of the invention in a collapsed configuration.
FIG. 20D illustrates a Y-construct flow diversion device according to one embodiment of the invention in an expanded configuration.
FIGS. 21A-C illustrates a process of shrinking a branch point aneurysm with the Y-shaped flow diversion device.
FIG. 22A illustrates an embodiment of a delivery device for a branch point aneurysm flow diversion device.
FIG. 22B illustrates a flow diversion device being deployed in a branch point aneurysm.
FIG. 22C illustrates the delivery device being open along a score line in to deploy the flow diversion device.
FIG. 22D illustrates the flow diversion device deployed to treat a branch point aneurysm.
FIG. 23A illustrates an embodiment of a delivery device for a branch point aneurysm flow diversion device.
FIG. 23B illustrates a distal containment stop decoupling from a distal containment stop of the delivery device.
FIG. 23C illustrates deploying the flow diversion device to treat a branch point aneurysm.
FIG. 23D illustrates the flow diversion device deployed to treat a branch point aneurysm.
FIG. 24 illustrates an embodiment of a flow diversion device with a plurality of wire elements with ovoid ring shapes.

### Detailed Description

With reference to the drawings, this section describes particular embodiments and their detailed construction and operation. The embodiments described herein are set forth by way of illustration only and not limitation. The described features, structures, characteristics, and methods of operation may be combined in any suitable manner in one or more embodiments. In view of the disclosure herein, those skilled in the art will recognize that the various embodiments can be practiced without one or more of the specific details or with other methods, components, materials, or the like. For the sake of clarity and conciseness, certain aspects of components or steps of certain embodiments are presented without undue detail where such detail would be apparent to those skilled in the art in light of the teachings herein and/or where such detail would obfuscate an understanding of more pertinent aspects of the embodiments.

The present inventor has recognized some disadvantages relating to current flow diversion devices. For example, one disadvantage is that the very fine braids in the wire mesh tube of current devices make it challenging to deliver the device intracranially, usually requiring larger diameter and stiffer microcatheters to advance the collapsed stent device to the target region of the affected artery. In some circumstances, these large bore, stiffer microcatheters may be unstable and lead to inadvertent displacement of the flow diversion device into the aneurysm cavity. Moreover, once in place, the construction of such stent devices may make their expansion somewhat difficult, which may cause undesired effects, such as: (1) leading to blockage of normal blood flow to part of the brain, thereby threatening a stroke, and (2) creating undesirable space between the outer surface of the stent and the blood vessel interior or intima, thereby permitting side branches to create an endoleak, which may result in the undesirable maintenance of flow into the aneurysm cavity.

Another disadvantage of such current flow diversion devices is that positional instability of the device during deployment may cause the device to unexpectedly kick back into the aneurysm. In addition, such current devices may also create a large metal burden for the treated artery, which may increase the risk for thromboembolic events and require prolonged therapy or treatment.

As further described in detail below, certain embodiments described herein may be capable of achieving various advantages, including one or more of the following: (1) providing a flow diversion device with a streamlined design to simplify endovascular introduction and decrease forces placed on the delivery catheter; (2) providing such a flow diversion device constructed to promote expansion of the device once in position for improved flow; and (3) providing a flow diversion device that effectively diverts blood away from an aneurysm while maintaining sufficient blood flow to maintain patency of arterial side branches adjacent the aneurysm treatment site. Additional aspects and advantages will be apparent from the following detailed description of example embodiments, which proceeds with reference to the accompanying drawings.

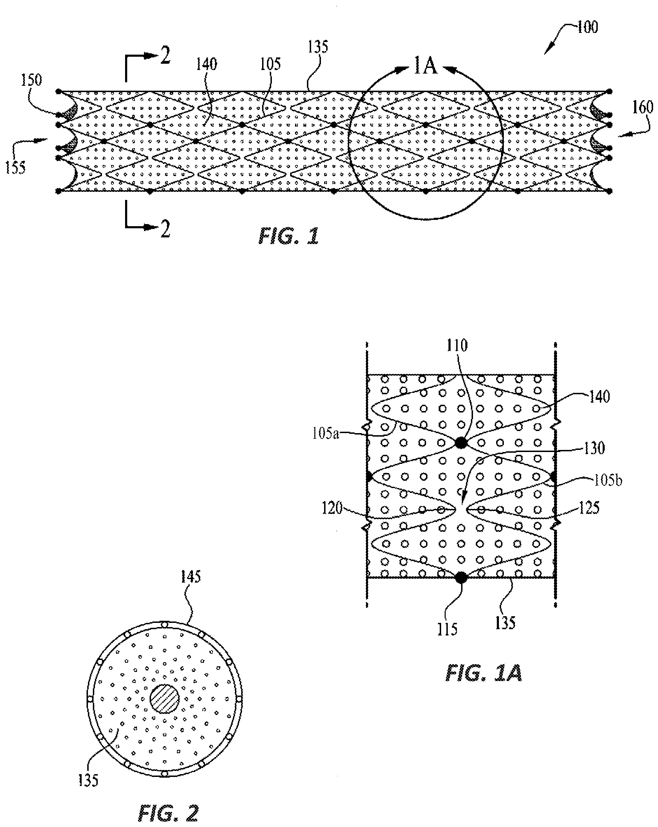

Collectively, FIGS. 1-14 illustrate various embodiments of a flow diversion device that may be used for the treatment of intracranial aneurysms and other medical procedures as further detailed below with reference to the figures. Generally, with reference to FIG. 1, the flow diversion device 100 is a stent-like device including a plurality of wire elements 105, where each individual wire element 105 is shaped in a zig-zag pattern and coupled to adjacent wire elements to form a general tube-shaped stent structure. The wire elements 105 hold open a thin base layer 135 and together form a support structure of the flow diversion device 100. An outer jacket 145 may surround the support structure formed of the wire elements 105 and the base layer 135, the jacket 145 being fused to the base layer 135 to provide added rigidity and maintain the tubular shape of the flow diversion device 100. As briefly mentioned previously, the flow diversion device 100 may be employed endovascularly to treat aneurysms by diverting blood flow away from the aneurysm to induce aneurysm thrombosis and for other suitable medical procedures. Additional details of these and other embodiments of the flow diversion device 100 are described herein with reference to the figures.

With particular reference to FIGS. 1 and 2, the following sections describe additional details of the flow diversion device 100 and the components thereof. As briefly described previously, the flow diversion device 100 includes a plurality of wire elements 105 coupled together to form a generally tubular-shaped, self-expanding stent structure. In some embodiments, the wire elements 105 may be composed of a metallic material exhibiting shape-memory/superelasticity qualities, such as Nitinol (a nickeltitanium alloy), Elgiloy (a cobalt, chromium, and nickel based alloy) or other suitable shape-memory alloys. In other embodiments, the wire elements 105 may instead be composed of other suitable materials, including non-metallic materials, capable of creating the tubular stent structure.

In some embodiments, the wire elements 105 may be coupled together, such as via a welding process, in a staggered or alternating pattern at one or more sites to create an "open cell" design capable of following sharp curves in blood vessels without kinking. For example, with particular reference to FIG. 1A, adjoining wire elements 105a and 105b may be welded at a first junction 110 and a second junction 115, leaving free at a central junction 130 a pair of ends 120, 125 of the respective wire elements 105a, 105b (i.e., the ends 120, 125 are not welded or otherwise attached to one another). In this configuration, the staggered welds between the adjacent wire elements 105a, 105b may help impart greater flexibility to the flow diversion device 100 without sacrificing the overall stability. It should be understood that while the embodiment of the flow diversion device 100 in FIG. 1 illustrates a welding pattern at alternating junctions, the wire elements 105 may instead be attached/linked in any one of a variety of suitable patterns. For example, in other embodiments, the wire elements 105 may be attached at every junction to increase stability, or may be attached in a non-alternating fashion at select junctions for additional flexibility.

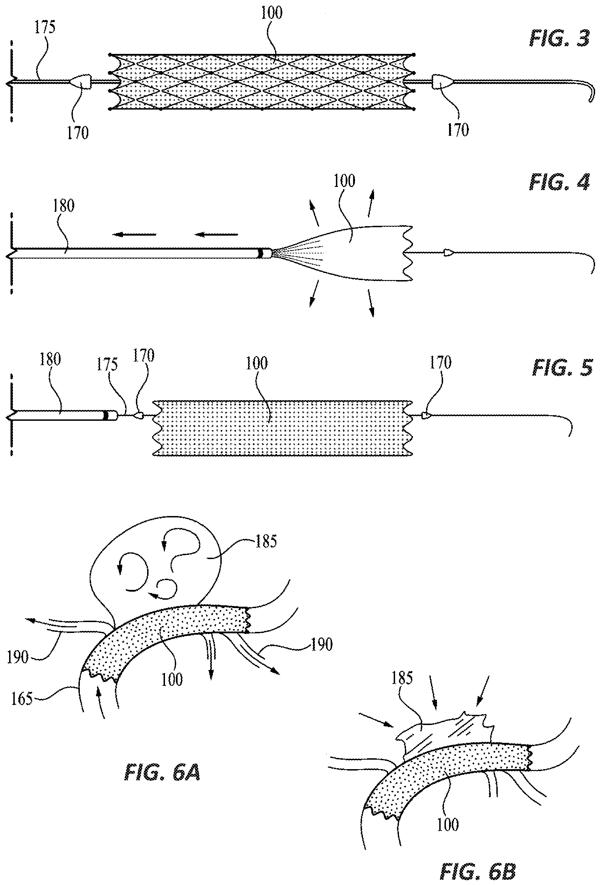

In some embodiments, the flow diversion device 100 may use or include few wire elements 105 to minimize resistance while the device 100 is advanced to a target site. In addition, the diameter of each of these wire elements 105 is selected to ensure each wire element 105 possesses sufficient expansive force once the flow diversion device 100 is deployed at the target site, thereby decreasing the chance of incomplete expansion, and in turn, avoiding a dangerous blockage of blood flow. For example, in one embodiment, the diameter of each wire element 105 may be equal to or less than 0.005 inches (=0.0127 cm) to create a flow diversion device 100 with sufficient stiffness, flexibility, and proper expansile forces to help ensure that the flow diversion device 100 is securely fixed to the target vessel site to cover the neck of an aneurysm or provide a flow pathway through a fusiform aneurysm as further described with particular reference to FIGS. 6A and 6B. In other embodiments, the diameter of each wire element 105 may be less than 0.010 inches (=0.0254 cm).

With reference to FIGS. 1 and 2, the flow diversion device 100 includes a tubular, ultra-thin base layer 135 of graft material, such as polytetrafluoroethylene (PTFE), or other suitable graft fabric to promote the flow diversion characteristics of the device 100. The base layer 135 and the stent frame formed of wire elements 105 may be attached to one another by any suitable means, such as via suture material, crimping, adhesive, or perhaps sealing one ultra-thin layer of graft material to another with the wire stent structure contained between the two layers 135, 145. To help ensure flow to and long-term patency of important small side branches frequently found along intracranial arteries, the graft material of the base layer 135 may include a plurality of minute perforations/pores 140 spaced apart on the base layer 135 to allow a small amount of blood flow through to the side branches. The perforation/pores 140 may be created mechanically, by using laser energy, or by any other suitable means. The size and shape of the perforations 140 may be designed so as to promote increased through-flow "on demand" (e.g., the pores allow greater flow to patent side branches but afford less flow to an aneurysm) without being too large so as to reduce the structural integrity of the base layer 135.

In some embodiments, the perforations/pores 140 may be designed and cut on the base layer 135 such that the perforations/pores 140 enlarge or change shape with increased flow demand. For example, the perforations/pores 140 may be half-circle incisions or slits, in a similar pattern as is typically found in a flag. As the blood flow against the base layer 135 increases, the half-circle incisions allow a flap of graft fabric to fold away (relative to the direction of blood flow) and prevent excess billowing of the base layer 135 (which might result in its tearing or dislodgement). Preferably, in the absence of any special characteristics of the perforations/pores 140, the porous graft material of the base layer 135, nonetheless, provides flow diversion by greatly inhibiting flow into an aneurysm sac, causing its gradual shrinkage and occlusion, while still providing diminished but sufficient flow to maintain patency of side branches.

As illustrated in FIG. 1, the flow diversion device 100 may include radiopaque markers 150 at both the proximal 155 and distal 160 ends. Using fluoroscopy, the radiopaque markers 150 allow an operator to view a position of the flow diversion device 100 and to determine whether the flow diversion device 100 has properly engaged the interior wall of the blood vessels or other lumen. In other embodiments, the wire elements 105 may also (or alternatively) include radiopaque material so that the periphery/boundary of the stent frame may be viewed.

With particular reference to FIG. 2, the base layer 135 of graft material may be fused with the outer jacket 145 to maintain the overall shape of the flow diversion device 100. In some embodiments, the jacket 145 may be made of polyurethane or other suitably strong, lightweight, and flexible polymer.

FIGS. 3-5 illustrate example embodiments for delivering the flow diversion device 100 to a blood vessel or other lumen 165 (see FIG. 6A). With particular reference to FIGS. 3 and 4, the flow diversion device 100 may be advanced in a compressed state between two metal bumpers 170 on a pusher/delivery wire 175 through a delivery catheter 180. Once the flow diversion device 100 is pushed to the target segment of the intracranial artery or other lumen 165 via the catheter 180, the catheter 180 may be withdrawn to allow the device 100 to expand at the target site. In other embodiments, the flow diversion device 100 may instead be mounted on a pusher wire and once the device is expanded/deployed at the target site; it can then be detached from the pusher wire by electrolytic or mechanical means. Both of the aforementioned methods of device delivery and deployment may, up to a certain point, allow retrieval of the flow diversion device if the deployed position is unsatisfactory.

FIGS. 6A and 6B schematically illustrate an example flow diversion process of the deployed device 100 when used to treat intracranial aneurysms. With reference to FIGS. 6A and 6B, the flow diversion device 100 is positioned adjacent an intracranial aneurysm 185 (such as a large or giant, wide-neck or fusiform intracranial aneurysm). When in position, the porous graft material of the base layer 135 reduces/inhibits blood flow into the aneurysm 185 thereby causing it to gradually occlude over time (as illustrated in FIG. 6B). Since the graft material is porous, a small amount of blood may flow into the aneurysm 185, but this flow is sufficiently small such that the aneurysm 185 will likely not continue growing. Moreover, the small amount of flow across the graft material remains sufficient to maintain patency of nearby side branches 190. In some treatments, for approximately a week prior to and three to six months following the deployment of the described flow diversion device 100, the patient may be treated with dual anti-platelet therapy (such as aspirin and clopidogrel (Plavix)). A follow-up cerebral angiogram may be performed at six and 12 months following device deployment to assess for aneurysm shrinkage and occlusion. If the aneurysm 185 is fully occluded and determined to be no longer problematic, the device 100 may be removed. In other instances, the device 100 may be left in position permanently to avoid potential future problems.

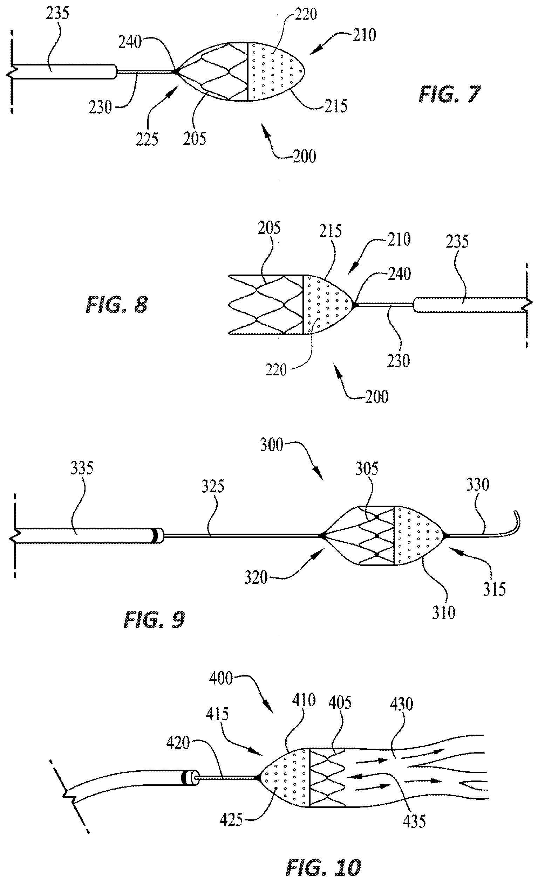

FIGS. 7-14 collectively illustrate various other embodiments for medical procedures and processes utilizing a similar concept of a wire mesh frame and a graft material base layer as described above with reference to the flow diversion device 100 of FIGS. 1-6. The following describes additional detail of these embodiments.

FIGS. 7 and 8 illustrate example embodiments of an inferior vena cava (IVC) filter 200 for helping prevent pulmonary emboli. With reference to FIG. 7, the IVC filter 200 may include a plurality of wire elements 205 each having a zig-zag shape, where each wire element 205 is welded or otherwise coupled to an adjacent element to form a stent frame in a similar fashion as described with respect to wire elements 105 of flow diversion device 100. The wire elements 205 at a distal end 210 of the IVC filter 200 may be gathered together and welded or otherwise mechanically constrained together to create a closed, conical shape at the distal end 210. A porous base layer 215 of graft material (e.g., PTFE, polyurethane or other suitable material) having perforations 220 that may range in diameter from 25 microns to 100 microns may be attached or otherwise mounted to the mesh structure of wire elements 205 along at least the distal end 210 portion. A proximal end 225 of the IVC filter 200 includes a tubular configuration that may exert sufficient expansive force so as to act as an endovascular "anchor."

With particular reference to FIG. 7, in some embodiments, the proximal end 225 of the IVC filter 200 may be attached to a guidewire 230 that, in conjunction with a delivery catheter 235, may be used to position the IVC filter 200 in the inferior vena cava. Positioning the guidewire 230 at the proximal end 225 may be useful for inserting the IVC filter 200 via the femoral vein so that the porous graft material is in the proper location relative to the direction of blood flow in the interior vena cava. With reference to FIG. 8, in other embodiments, the distal end 210 of the IVC filter 200 may instead be attached to the guidewire 230 and delivered to the inferior vena cava, such as via the internal jugular vein. In both embodiments, the IVC filter 200 may be a temporary filter (where the proximal or distal wire could be used as a means for retrieval of the IVC filter 200), or may instead be a permanent IVC filter 200. In such embodiments where the IVC filter 200 is permanent, the IVC filter 200 may include a detachment zone 240 positioned between the introduction guidewire 230 and the IVC filter 200 for allowing detachment of the IVC filter 200 when desired. Alternatively, the guidewire 230 may be used to slide a recover/resheathing catheter over the IVC filter 200 and retrieve it if desired.

In use, the stent-like body (with a closed cell design) composed of the welded wire elements 205 make the filter self-centering within the interior vena cava. In addition, the perforated graft material of the base layer 215 is positioned to accommodate blood flow and otherwise trap thromboemboli from the pelvis and/or lower extremities that could otherwise cause a fatal pulmonary embolus if it reached the lungs. Such an IVC filter 200 may be advantageous since PTE (pulmonary thromboemboli) is currently the third leading cause of death in the United States.

FIG. 9 illustrates an example embodiment of an anchor-filter device 300 that may be used for carotid, coronary, bypass graft angioplasty, and stenting. With reference to FIG. 9, the anchor-filter device 300 may include a similar stent-like scaffold made of welded wire elements 305 and a porous base layer 310 attached to a distal end 315 of the anchor-filter device 300 in a similar fashion as described with reference to the IVC filter 200 of FIGS. 7 and 8. A proximal end 320 of the anchor-filter device 300 may be attached to a guidewire 325 (such as a 0.014" to a 0.018" proximal working wire). The guidewire 325 may run the length of the anchor-filter device 300 and emerge distally as a short shapeable wire extension 330 for accommodating navigation past a stenosis and into the more normal distal arterial segment while the anchor-filter device 300 is in a collapsed state in a microcatheter 335 (approximately 3-F outer diameter). Such an anchor-filter device 300 may help prevent distal debris embolization arising from percutaneous transluminal angioplasty (PTA) and stenting of an atherosclerotic vessel by filtering procedural debris. Once the procedure is completed, the anchor-filter device 300 is collapsed by the microcatheter 335 slid up along the proximal guidewire 325, whereupon the device 300 would be removed from the artery.

In cases of extremely tortuous great vessel anatomy of the thoracic aortic arch (such as in the elderly and hypertensive), proximal access within the innominate or left common carotid artery could be established with a stiffer (e.g., ALZ) 7-F coronary guiding catheter, and then an approximately 2.8-F delivery microcatheter can be guided through the high-grade ICA stenosis over a 0.014" wire. This wire may then be removed and replaced with the anchor-filter device that would then be deployed in the upper cervical ICA. Here it would stabilize the working wire and then permit easier, more rapid catheterizations in thoracic arch tortuous anatomy by means of a bi- or triaxial catheter system, and may potentially also be useful to prevent distal grumous embolization during angioplasty and stenting of stenotic coronary bypass grafts. Once debris is captured at the conclusion of PTA and stenting, the filter device is re-captured by advancing an approximately 2.8-F recovery catheter over the working wire (which was used to also introduce PTA balloon catheters and stent devices) and collapsing the filter.

In still other embodiments, with reference to FIG. 10, the combination of perforated graft material and stent-like scaffold may be used for a reverse or retrograde filter device 400 to provide a mechanism for infusing chemotherapeutic agent-soaked embolic particles or minute Y⁹⁰ radio-embolization particles. With reference to FIG. 10, the retrograde filter device 400 may include a wire mesh scaffold comprising a plurality of wire elements 405 attached in a similar fashion as described previously with respect to the devices 100, 200, 300. In addition, the retrograde filter device 400 further includes a base layer 410 of perforated graft material (e.g., PTFE or polyurethane) mounted or otherwise attached around a proximal end 415 of the wire mesh frame of the retrograde filter device 400.

The proximal end 415 of the wire mesh frame comprising wire elements 405 is mounted to a flexible hypotube 420 having an inner diameter of approximately 0.014" or any other inner diameter suitable to allow the injection of particles (e.g., Biospheres, polyvinyl alcohol particles, Y⁹⁰TheraSphere particles) for radioembolization. In the case of radioembolization, backflow of the injected particles from the intra-arterial point of embolization may cause the particles to flow into a vital collateral channel serving the small bowel or stomach (e.g., the right gastric artery-in this case the radioactive Y⁹⁰ particles could induce a nonhealing ulcer of the gastric mucosa). To avoid such problematic backflow, the proximal end 415 of the wire mesh stent has a conical shape and the perforations 425 on the graft material of the base layer 410 are appropriately sized to allow antegrade flow within a target vessel 430, but not to allow the backflow of injected particles, thus avoiding nontarget embolization into more proximal branch or collateral vessels. As illustrated in FIG. 10, a distal end 435 of the wire mesh stent may be open and have a cylindrical or tubular shape, the distal end 435 not being entirely covered with the graft material of the base layer 410. Accordingly, the distal end 435 fully expands to bear against the target vessel's inner walls and maintain the retrograde filter device 400 firmly in position.

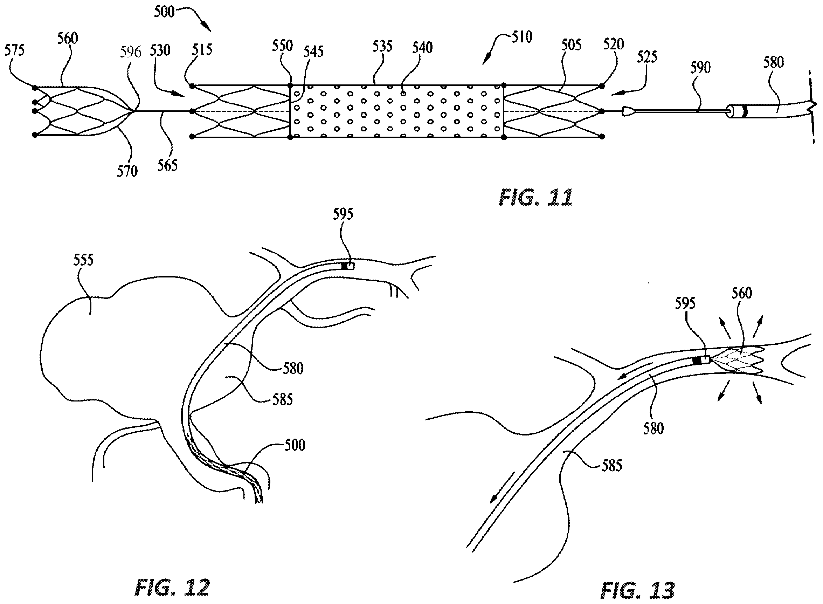

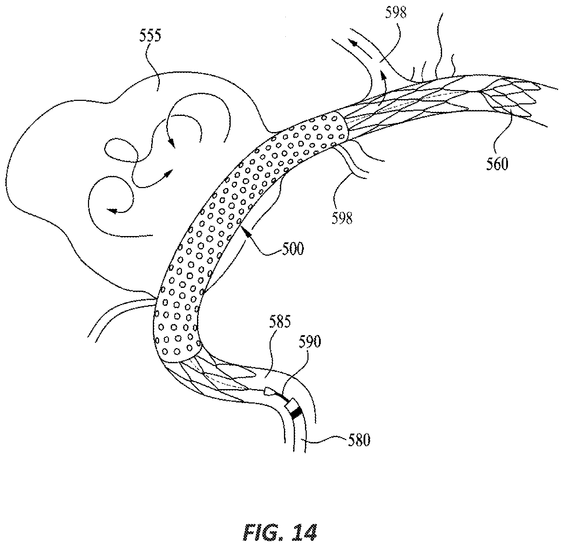

FIG. 11 illustrates another example embodiment of a flow diversion device 500 that may be used to divert blood flow away from an aneurysm, while also maintaining sufficient blood flow to maintain patency of large side branches 598 (see FIG. 14). With reference to FIG. 11, the flow diversion device 500 includes a self-expanding, partially open-cell stent frame 510 formed from a plurality of wire elements 505 coupled in a similar fashion as described with respect to the flow diversion device 100 of FIG. 1. In some embodiments, the flow diversion device 500 may include radiopaque markers 515, 520 on the wire elements 505 at both a proximal end 525 and a distal end 530 of the stent frame 510 to allow an operator to monitor a position of the flow diversion device 500 using fluoroscopy. In other embodiments, the wire elements 505 may also (or alternatively) include radiopaque material so that the periphery or boundary of the stent frame 510 may be monitored during the insertion and treatment procedure.

The flow diversion device 500 includes an ultra-thin base layer 535 of graft material, such as polytetrafluoroethylene (PTFE), or other suitable graft fabric that surrounds at least a portion of the stent frame 510, with the base layer 535 including a plurality of perforations/pores 540 formed thereon to help control blood flow to the treatment site. Preferably, the base layer 535 is positioned on a substantially central portion of the stent frame 510, leaving the proximal and distal ends 525, 530 free of the base layer 535. As is explained in additional detail below, the base layer 535 inhibits or reduces blood flow therethrough due to the relatively small size of the perforations 540, but the openness of the stent frame 510 and the proximal and distal ends 525, 530 allow for increased blood flow to large side branches 598 that may be adjacent the treatment site of the aneurysm 555. In some embodiments, a portion of a boundary 545 of the base layer 535 may include radiopaque markers/material 550 to allow an operator to precisely place the stent frame 510 at a particular treatment site and to ensure that blood flow away is directed away from the aneurysm 555 while also directing sufficient blood flow to large side branches 598 adjacent the aneurysm 555 as further described in detail below with reference to FIGS. 12-14.

In some embodiments, the flow diversion device 500 may include an anchor stent 560 attached to a guidewire 565 and extending forwardly from the distal end 530 of the stent frame 510. With reference to FIG. 11, the anchor stent 560 is a generally tubular-shaped, self-expanding mesh structure similar in construction to the stent frame 510. The anchor stent 560 includes a plurality of connector struts 570 coalescing to a strut tip 596 at which the guidewire 565 is attached. In some embodiments, the anchor stent 560 may also include radiopaque markers 575 on an end opposite the connector struts 570. In use, the anchor stent 560 provides a means to secure in place the flow diversion device 500 to ensure proper treatment is provided for the aneurysm 555.

FIGS. 12-14 illustrate example embodiments for delivering the flow diversion device 500 to a target site for treating an aneurysm 555. With general reference to FIGS. 12-14, a delivery catheter 580 is advanced into a blood vessel 585 beyond the treatment site where the aneurysm 555 is located. As illustrated in FIG. 12, once the catheter 580 is in position, the flow diversion device 500 is advanced via a guidewire 590 through the catheter 580, the flow diversion device 500 being in a compressed state as it travels through the catheter 580. Once the flow diversion device 500 reaches the end 595 of the catheter 580, the anchor stent 560 extends outwardly from the end 595 and expands radially against an inner wall of the vessel 585 to stabilize the catheter 580 as illustrated in FIG. 13. Once the anchor stent 560 is fully expanded, the catheter 580 may be retracted away from the target site, wherein retracting the catheter 580 unsheathes the flow diversion device 500.

In one example deployment process, the guidewire 590 is held stationary while the catheter 580 is retracted, with the anchor stent 560 helping retain the flow diversion device 500 in position at the target site. With reference to FIG. 14, once the catheter 580 is fully retracted, the flow diversion device 500 is at the target position, with the base layer 535 adjacent the aneurysm 555 to divert blood away from the aneurysm 555 and cause the aneurysm 555 to occlude over time. In addition, as mentioned previously, the open proximal and distal ends 525, 530 of the stent frame 510 provide for increased blood flow to larger side branches 598 that may be near the aneurysm 555, and the perforations 540 on the base layer 535 provide sufficient flow for the smaller side branches 598.

When the treatment is complete and the flow diversion device 500 is ready for removal, the flow diversion device 500 may be resheathed by advancing the catheter 580 toward the proximal end 525 of the stent frame 510. As the catheter 580 moves forward, the flow diversion device 500 collapses into the catheter 580. Once the entire device 500 is sheathed in the catheter 580, the catheter 580, together with the device 500, may be removed.

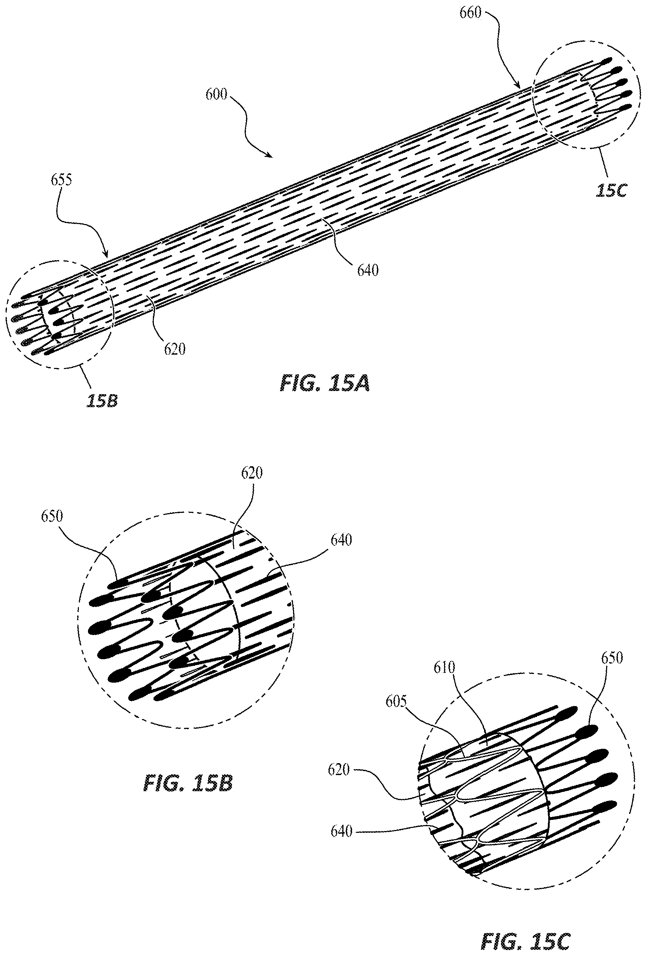

FIGS. 15A-16 illustrate exemplary flow diversion devices in a collapsed configuration and an expanded configuration. FIG. 15A illustrates a flow diversion device 600 with a similar stent-like scaffold made of welded wire elements 605 with a zig-zag pattern discussed previously. The flow diversion device 600 may further include a base layer 610 of graft material, such as PTFE, or other suitable graft fabric. The base layer 610 may be attached to the stent frame by any suitable means, such as via suture material, crimping, or adhesive. In some embodiments, the base layer 610 may be sealed to outer layer 620 with the wire stent structure contained between the two layers 610 and 620, as illustrated in FIG. 15B.

As illustrated in FIGS. 15B and 15C, the flow diversion device 600 may include radiopaque markers 650 at both the proximal 655 and distal 660 ends. In the illustrated embodiment, the welded wire elements 605 may extend past the base layer 610 on the proximal 655 and distal 660 ends. Using fluoroscopy, the radiopaque markers 650 allow an operator to view a position of the flow diversion device 600 and to determine whether the flow diversion device 600 has properly engaged the interior wall of the blood vessels or other lumen. In other embodiments, the wire elements 605 may also (or alternatively) include radiopaque material so that the periphery/boundary of the stent frame may be viewed.

The expansion of the stent-like scaffold of the flow diversion device 600 may be aided by multiple slits/fenestrations 640 disposed in the base layer 610. In embodiments that include a base layer 610 and an outer layer 620, the slits 640 on the base layer 610 and the outer layer 620 may align with each other. The slits 640 may be longitudinally arranged, i.e., aligning with the longitudinal axis of the flow diversion device 600. The slits 640 may have a length of less than 1 mm.

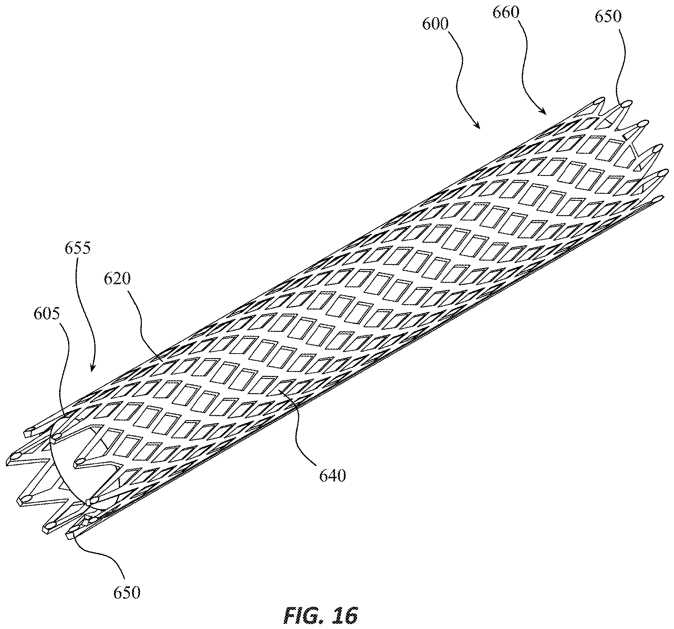

In a compressed configuration, the slits 640 are closed and are opened during expansion of the flow diversion device 600. For example, FIG. 16 illustrates the flow diversion device 600 in an expanded configuration and the slits 640 have expanded to a rhombus or diamond shape. The expanded slits 640 allow a small amount of blood flow through base layer 610 to the side branches to help ensure flow to and long-term patency of small side branches frequently found along intracranial arteries. However, most blood flow would be diverted past the aneurysm causing the gradual shrinkage and occlusion of the aneurysm. The slits/fenestrations 640 may be created mechanically, by using laser energy, or by any other suitable means. The size and shape of the slits 640 may be designed so as to promote increased through-flow "on demand" (e.g., the pores allow greater flow to patent side branches but afford less flow to an aneurysm) without being too large so as to reduce the structural integrity of the base layer 610.

As discussed previously, the flow diversion device may include an inner layer (e.g., base layer 610) and an outer layer (e.g., outer layer 620) of graft material with the scaffold (e.g., welded wire elements 605) in-between. The longitudinal slits 640 may match on the inner and outer layers of the graft material. The flow diversion device 600 may also be a single base layer attached to the stent with longitudinal slits.

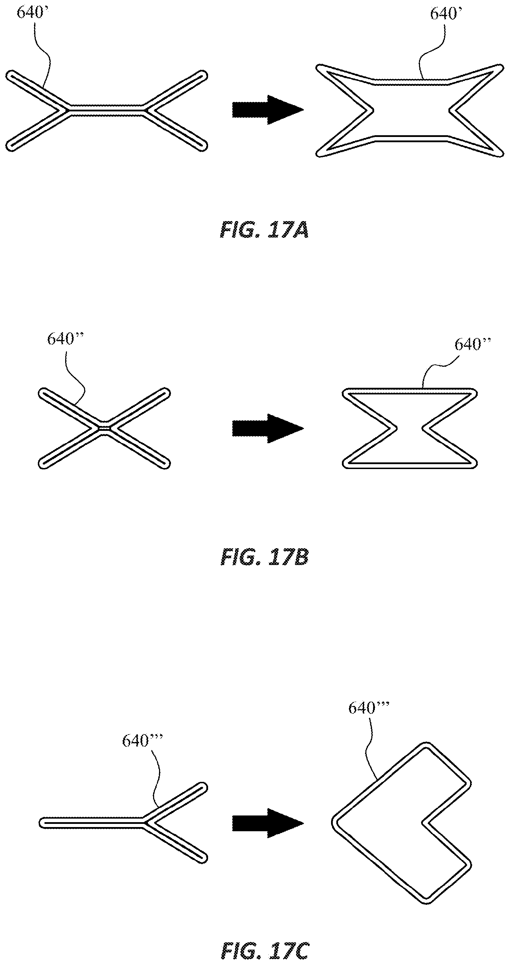

In addition to straight slits that expand to diamond-shape (or rhomboid-shape) fenestrations, the expandable slits may be produced in a variety of different shapes. FIGS. 17A-17C illustrate alternative slit designs. For example, FIG. 17A illustrates slits 640' with opposing Y-shaped slit in a collapsed configuration that expands to an expanded hourglass-shaped fenestration in an expanded configuration. FIG. 17B illustrates slits 640" with an X-shape slit in a collapsed configuration that expands to an hourglass-shaped fenestration in an expanded configuration. FIG. 17C illustrates slits 640‴ with a Y-shaped slit in a collapsed configuration that expands to a V-shaped fenestration in an expanded configuration.

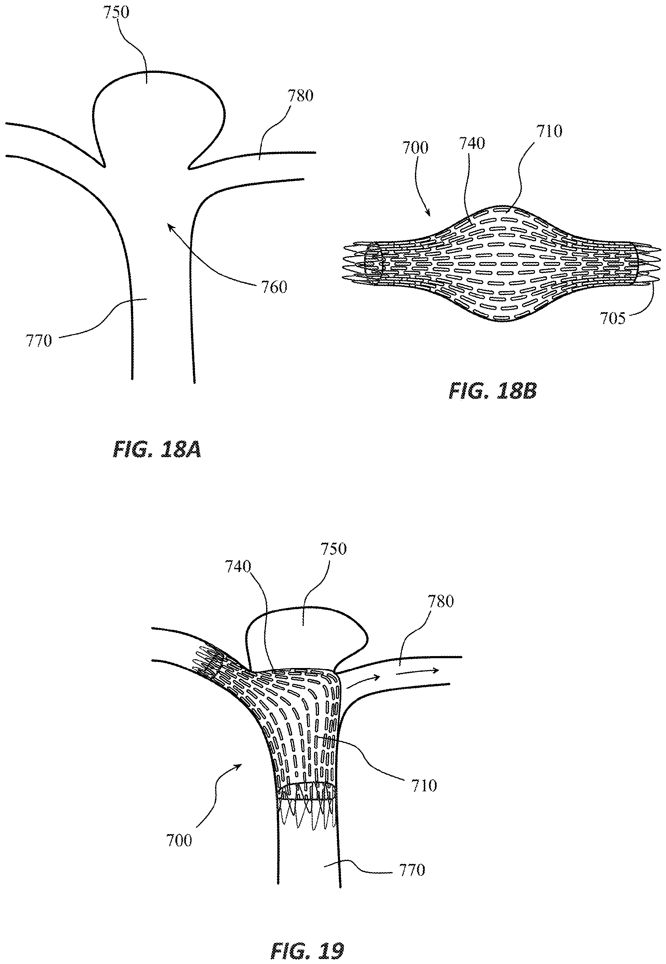

FIGS. 18A-18B and 19 illustrate an exemplary embodiment of a flow diversion device 700 that is configured to apply flow diversion for aneurysms 750 that occur at branch points in arteries, such as brain arteries. FIG. 18A illustrates a exemplary branch point 760. FIG. 18B illustrates the flow diversion device 700 with a scaffold stent 705 that includes a bulge or barrel-like segment 710 in a central portion of the scaffold stent 705. The flow diversion device 700 may fully cover the entry to the aneurysmal cavity 750. The bulge 710 may expand to greater dimensions needed for a vessel lumen at the branch point 760. FIG. 19 illustrates the flow diversion device 700 placed within a blood vessel 770 at the branch point 760. The flow diversion device 700 causes the aneurysm 750 to shrink and occlude over a period of time. The flow diversion device may include a plurality of slits/fenestrations 740 similar to the slits discussed in regard to FIGS. 15A-17C. The slits/fenestrations 740 of the expanded flow diversion device 700 may enable blood flow to a side branch 780.

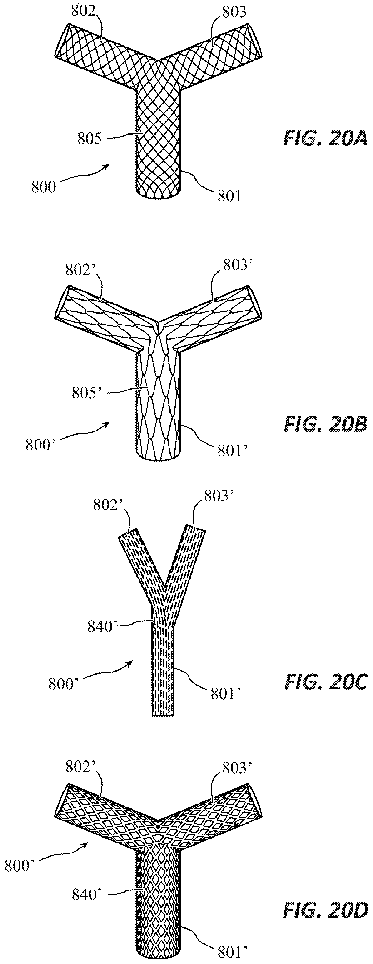

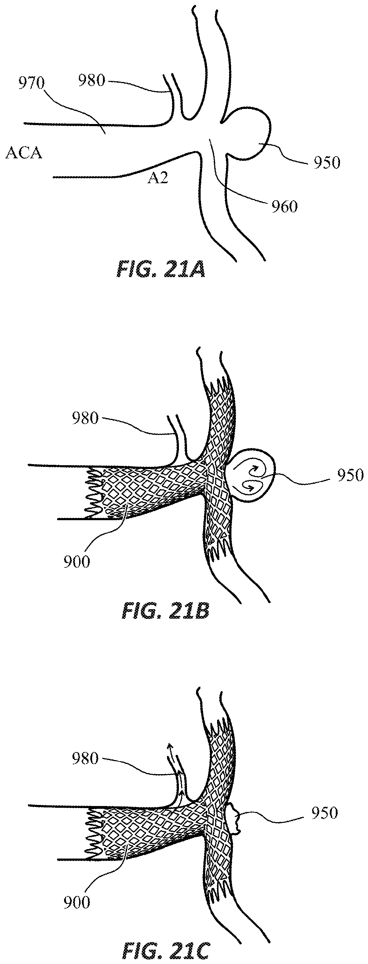

FIGS. 20A-20D illustrate embodiments of branch point wide-neck aneurysm flow diversion devices. The flow diversion devices of FIGS. 20A-20D may be applied to arterial branch points, such as brain arteries. FIG. 20A illustrates a flow diversion device 800 may be Y-shaped having a proximal limb 801 and two distal limbs 802 and 803. The Y-shaped flow diversion device 800 may be in the form of a braided wire device with a plurality of wire elements 805. The braided wire design for the Y-construct may be a self-expanding woven Nitinol wire design with a myriad of tiny closed wire cells with less than 0.5 mm cell diameter that would be small enough to create stagnation of flow within a branch point aneurysm but would allow just enough flow to keep side branches and perforators patent. The wire elements 805 may be fabricated between 0.0008- and 0.0017-inch (= 0.002032 and 0.004318 cm) diameter having about 75% chromium-cobalt alloy and 25% platinum-tungsten allow wires. Each limb 801, 802, and 803 may each have 48 to 60 wires. Two of the limbs 802 and 803 of the flow diversion device 800 may extend underneath a wide-neck saccular aneurysm into distal branch vessels (see FIGS. 21A-21C). The proximal limb 801 of the flow diversion device 800 may anchor the flow diversion device 800 within the proximal parent vessel of the branch point aneurysm. The Y-shaped flow diversion device 800 may be self-expanding.

FIGS. 20B-20D illustrate a Y-shaped flow diversion device 800' comprising a plurality of wire elements 805' that form a zig-zag self-expanding wire stent (fabricated from Nitinol, for example, and having a wire diameter of approximately 0.001 inches (= 0.00254 cm)). The plurality of wires 805' may act as a scaffold for an ultra-thin branched tubular layer of PTFE. FIG. 20C illustrates the Y-shaped flow diversion device 800' in a collapsed configuration. The flow diversion device 800' may include multiple tiny slits/fenestrations 840' disposed in the ultra-thin branched tubular layer 810'. FIG. 20D illustrates the Y-shaped flow diversion device 800' in an expanded configuration so that once the flow diversion device 800' is fully expanded, the slits 840' may create diamond-shaped fenestrations within the PTFE layer 810'. These slits/fenestrations 840' (<0.5 mm in diameter) may create enough flow restriction to the aneurysm cavity to create stagnation of low and eventual occlusion. In some embodiments, the Y-shaped flow diversion device may be expanded by a balloon, e.g., the device may be composed of multiple zig-zag wire ring elements that when the balloon expanded would create a myriad of minute interstices in-between the wire zig-zags that are small enough to promote flow diversion. The distal flow diverter limbs may be crimped-down onto separate ultralow profile balloon catheters that would proximally coalesce into a single balloon catheter onto which may be crimped the proximal balloon-expandable stent. The two distal balloon-expandable limbs may be advanced over separate 0.014" guidewires into the arterial limbs composing the branch point. Once in position, all three stent limbs may be balloon expanded. Such a balloon-expandable Y-shaped flow diverter may also be mounted with a fenestrated ultra-thin PTFE tubular layer that may also act to create flow diversion while maintaining patency of side branches and perforators.

FIGS. 21A-21C illustrate the process of shrinking a branch point aneurysm with a Y-shaped flow diversion device 900. FIG. 21A illustrates an aneurysm 950 located at a branch point 960 of a vessel 970. FIG. 21B illustrates the flow diversion device 900 deployed and expanded at the branch point 960. The flow diversion device 900 may stagnate the flow of blood to the aneurysm 950, but maintain patency of side branches 980. After a period of time, such as six months, the aneurysm 950 may shrink as illustrated in FIG. 21C.

One example of the use of such a Y-shaped flow diversion device may be a wide-neck aneurysm at the junction among the A2 segment of the anterior cerebral artery, the origin of the internal frontal branches, and the pericallosal artery. Once the two distal flow diverter limbs are positioned at the origin of the internal frontal branches and the pericallosal artery, the proximal limb is deployed within the A2 segment. Flow diversion may lead to flow stagnation within the aneurysm cavity (a dead-end sack) and eventual involution of this structure. Other examples where use of the Y-shaped flow diversion device may be useful would be a basilar tip of an ICA terminus wide-neck aneurysm.

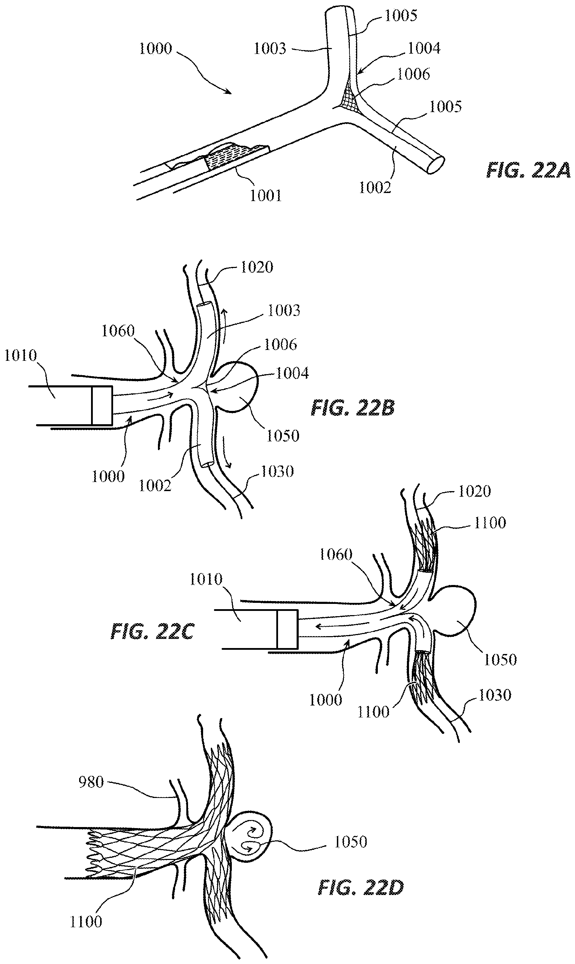

In some embodiments, the flow diversion device may be advanced to a deployment site via a peel-away Y-shaped sheath 1000, as illustrated in FIGS. 22A-22D. The Y-shaped sheath 1000 may include a proximal limb 1001 and two distal limbs 1002 and 1003. The Y-shaped sheath 1000 may further possess a scored line 1005 along the inner surfaces of its distal limbs 1002 and 1003 that meet at the crotch 1004 of the "Y" of the sheath 1000. The crotch 1004 of the Y-shaped sheath 1000 further includes a small hole or fenestration 1006. FIG. 22B illustrates the Y-shaped sheath 1000 being inserted into a branch point 1060 of a vessel to treat an aneurysm 1050. The Y-shaped sheath 1000 may be deployed to the branch point 1060 through the use of guidewires 1020 and 1030. FIG. 22C illustrates the Y-shaped sheath 1000 being retracted and removed into a catheter 1010 to deploy a flow diversion device 1100. The scored line 1005 is separated or torn when the sheath 1000 is withdrawn in the proximal direction into the catheter 1010. In some embodiments, the scored line 1005 may be aided in separation by an electrolytic mechanism. Once the Y-shaped sheath 1000 is withdrawn into the catheter 1010, the flow diversion device 1100 may be left in place at the branch point 1060 to treat the aneurysm 1050.

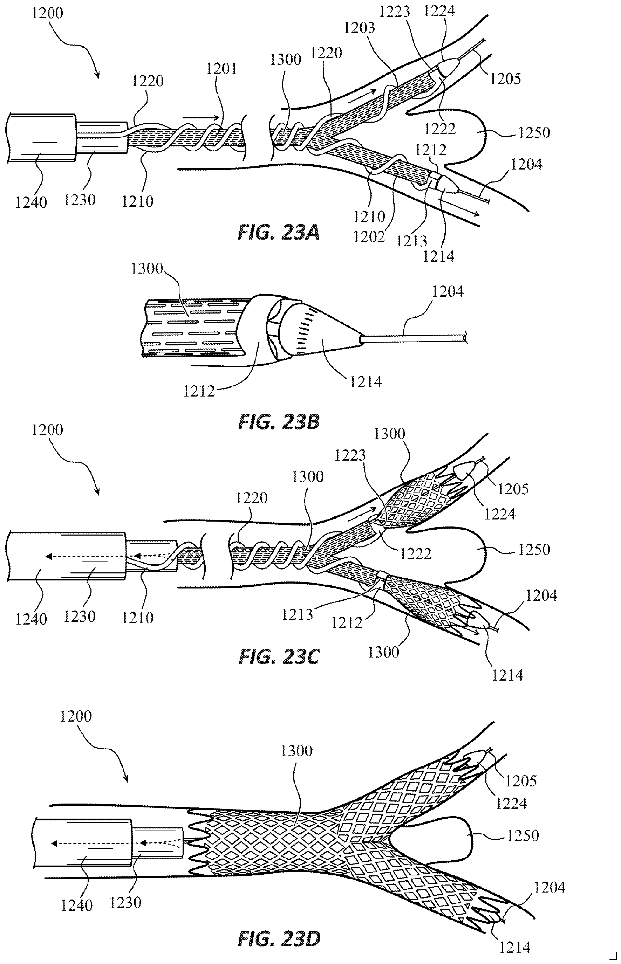

Alternatively, a self-expanding Y-construct flow diversion device 1300 could be introduced to a branch point aneurysm 1250 via a spiral containment strand system 1200, as illustrated in FIGS. 23A-23D. The spiral containment strand system 1200 may be housed within a delivery catheter 1240 and a pusher catheter 1230 may be used to help deploy the flow diversion device 1300 and the spiral containment strand system 1200. The spiral containment strand system 1200 may include a plurality of guidewires 1204 and 1205. The flow diversion device 1300 may be advanced over the guidewires 1204 and 1205. The spiral containment strand system 1200 may include flexible hypotubes or spiral containment bands 1210 and 1220, made from polyvinyl alcohol (PVA), nylon, or Teflon, etc. The spiral containment bands 1210 and 1220 may be wrapped around the Y-construct flow diversion device 1300 to constrain the flow diversion device 1300 in a collapsed configuration or low-profile state, permitting it to be advanced to a deployment position, as illustrated in FIG. 23A. A first spiral containment band 1210 may be wrapped around a proximal sheath limb 1201 and around a distal sheath limb 1202. A second spiral containment band 1220 may be wrapped around the proximal sheath limb 1201 and around a distal sheath limb 1203. The distal ends of the thin spiral containment bands 1210 and 1220 may include a distal containment rings 1212 and 1222 that are respectively coupled to distal containment stops (olives) 1214 and 1224 that constrict the self-expanding Y-construct flow diversion device 1300. The distal containment rings 1212 and 1222 may have a ring shape that encompasses the flow diversion device 1300. The distal containment stops 1214 and 1224 may include a lumen through which the distal containment stop may be coupled to the guidewires 1204 and 1205. As illustrated in FIG. 23B, the distal containment stops 1214 and 1224 may be separated from the distal containment rings 1212 and 1222 by either electrolytical or mechanical detachment. After detachment between the distal containment rings 1212 and 1222 and the distal containment stops 1214 and 1224, the spiral containment bands 1210 and 1220 may be pulled off of the Y-construct flow diversion device 1300 in a proximally oriented direction, allowing the Y-construct flow diversion device 1300 to expand, as illustrated in FIG. 23C. While the spiral containment bands 1210 and 1220 may be pulled in a proximally oriented direction, the distal containment rings 1212 and 1222 may open or break along a predetermined axial seam 1213 and 1223 when the distal containment rings 1212 and 1222 reach a crotch 1306 of the Y-construct. FIG. 23D illustrated the Y-construct flow diversion device 1300 in a deployed or expanded configuration.

In some embodiments, prior to deployment, the patient may be treated with oral aspirin and Plavix or another suitable anti-platelet drug regimen, and may be heparinized during the procedure. The insertion procedure may begin with an intermediate sized delivery catheter (4-4.5F, outer diameter) being advanced into the parent vessel just proximal to the branch point aneurysm. Next, 0.014-inch (= 0.03556 cm) diameter exchange guidewires may be advanced into the distal arterial limbs of the branch point. Over these exchange guidewires, the branch point flow diversion device (in its various iterations) would be advanced through the intermediate catheter until it is in satisfactory position adjacent to the osteum of the branch point aneurysm. The device is then deployed and its delivery catheters would be removed. Afterwards, the patient would be maintained on an anti-platelet drug regimen, e.g., aspirin and clopidogrel or Prasugrel. The patient may undergo a sixmonth cerebral angiogram to assess for aneurysm occlusion.

The self- or balloon-expandable Y-shaped flow diversion device made from braided or woven wires or myriad zig-zag wire rings may also be useful in other locations, such as the biliary tree, the branch point of the trachea (carina), and at sites of tumors. The Y-shaped flow diversion device may be coated with Yttrium and then placed in a nuclear reactor or other high-energy particle environment where the beta-emitting isotope Y⁹⁰ is created. Such a Y-construct may be used to combat both biliary obstruction (e.g., due to cholangiocarcinoma) and kill tumor cells with beta radiation. A branch point vascular stent impregnated in such a fashion with Y⁹⁰ may be useful to fight the development of in-stent stenosis using beta radiation.

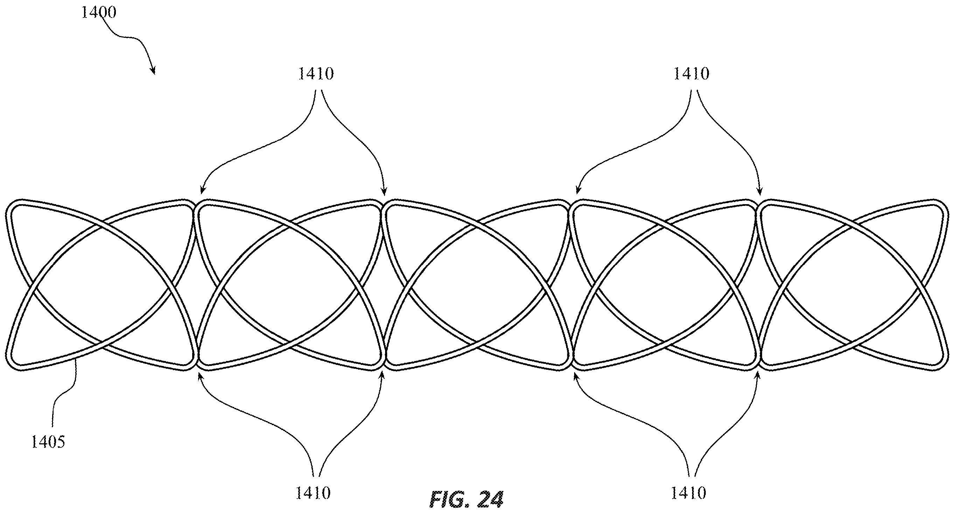

FIG. 24 illustrates an embodiment of a flow diversion device 1400 that is a stent-like device including a plurality of wire elements 1405, where each individual wire element 1405 is a shaped ovoid ring pattern and interwoven with other ovoid ring patterns. Each individual wire element 1405 may be coupled or welded to adjacent wire elements 1405 at coupling locations 1410 to form a general tube-shaped stent structure. The ovoid ring structure illustrated in FIG. 24 may be beneficial for flexibility purposed in Y-construct flow diversion devices previously discussed.

## Claims

1. A branched flow diversion device (800, 800') comprising:
a wire stent frame comprising a plurality of wire elements (805, 805'),
wherein the wire stent frame comprises a proximal limb (801, 801') and at least
two distal limbs (802,803; 802', 803'),
wherein the proximal limb (801, 801') and the at least two distal limbs (802, 803; 802', 803') converge at a crotch of the wire stent frame;
**characterized in that**
the plurality of wire elements (805, 805') are woven together and form a myriad of closed wire cells with less than 0.5 mm cell diameter.

2. The branched flow diversion device (800, 800') of claim 1, whereinthe plurality of wire elements (805, 805') forms a zig-zag self-expanding wire stent.

3. The branched flow diversion device (800, 800') of one of claims 1 or 2, wherein a diameter of the plurality of wire elements (805, 805') is equal to or less than 0.0127 cm, preferably 0.00254 cm.

4. The branched flow diversion device (800, 800') of one of claims 1 to 3, wherein the plurality of wire elements (805, 805') for each limb (801,802, 803; 801', 802', 803') of the wire stent frame comprises 48-60 wires.

5. The branched flow diversion device (800, 800') of one of claims 1 to 4, wherein the wire stent frame has a Y-shaped configuration.

6. (800, 800') The branched flow diversion device (800, 800') of one of claims 1 to 5, wherein each limb comprises a tubular shape and a lumen.

7. The branched flow diversion device (800, 800') of one of claims 1 to 6,wherein the wire frame stent is fabricated from Nitinol.

8. The branched flow diversion device (800, 800') of one of claims 1 to 7, further comprising a base layer attached to the wire stent frame andsurrounding at least a portion thereof, the base layer formed of a porous graft material having a plurality of slits formed in a longitudinal direction of the flow diversion device (800, 800'), wherein the slits have a different shaped in a compressed configuration and in an expanded configuration.

9. The branched flow diversion device (800, 800') of claim 8, wherein in the compressed configuration, the slits are straight and in the expanded configuration, the slits have a diamond shape or rhomboid shape.

## Patentansprüche

1. Verzweigte Strömungsumleitungsvorrichtung (800, 800'), umfassend:
einen Drahtstentrahmen, der eine Vielzahl von Drahtelementen (805, 805') umfasst,
wobei der Drahtstentrahmen einen proximalen Schenkel (801, 801') und mindestens zwei distale Schenkel (802, 803; 802', 803') umfasst,
wobei der proximale Schenkel (801, 801') und die mindestens zwei distalen Schenkel (802, 803; 802', 803') an einem Schritt des Drahtstentrahmens konvergieren;
**dadurch gekennzeichnet, dass**
die Vielzahl von Drahtelementen (805, 805') miteinander verwoben sind und eine Unzahl von geschlossenen Drahtzellen mit einem Zelldurchmesser von weniger als 0,5 mm bilden.

2. Verzweigte Strömungsumleitungsvorrichtung (800, 800') nach Anspruch 1, wobei die Vielzahl von Drahtelementen (805, 805') einen selbstexpandierenden Zickzack-Drahtstent bildet.

3. Verzweigte Strömungsumleitungsvorrichtung (800, 800') nach einem der Ansprüche 1 oder 2, wobei ein Durchmesser der Vielzahl von Drahtelementen (805, 805') gleich oder kleiner als 0,0127 cm, vorzugsweise 0,00254 cm ist.

4. Verzweigte Strömungsumleitungsvorrichtung (800, 800') nach einem der Ansprüche 1 bis 3, wobei die Vielzahl von Drahtelementen (805, 805') für jeden Schenkel (801, 802, 803; 801', 802', 803') des Drahtstentrahmens 48-60 Drähte umfasst.

5. Verzweigte Strömungsumleitungsvorrichtung (800, 800') nach einem der Ansprüche 1 bis 4, wobei der Drahtstentrahmen eine Y-förmige Konfiguration aufweist.

6. Verzweigte Strömungsumleitungsvorrichtung (800, 800') nach einem der Ansprüche 1 bis 5, wobei jeder Schenkel eine röhrenförmige Form und ein Lumen umfasst.

7. Verzweigte Strömungsumleitungsvorrichtung (800, 800') nach einem der Ansprüche 1 bis 6, wobei der Drahtrahmenstent aus Nitinol hergestellt ist.

8. Verzweigte Strömungsumleitungsvorrichtung (800, 800') nach einem der Ansprüche 1 bis 7, ferner umfassend eine Basisschicht, die an dem Drahtstentrahmen angebracht ist und mindestens einen Abschnitt davon umgibt, wobei die Basisschicht aus einem porösen Transplantatmaterial gebildet ist, das eine Vielzahl von Schlitzen aufweist, die in einer Längsrichtung der Strömungsumleitungsvorrichtung (800, 800') gebildet sind, wobei die Schlitze in einer komprimierten Konfiguration und in einer expandierten Konfiguration eine unterschiedliche Form aufweisen.

9. Verzweigte Strömungsumleitungsvorrichtung (800, 800') nach Anspruch 8, wobei in der komprimierten Konfiguration die Schlitze länglich sind und in der expandierten Konfiguration die Schlitze eine Diamantform oder Rhomboidform aufweisen.

## Revendications

1. Dispositif de déviation d'écoulement ramifié (800, 800') comprenant :
un cadre de stent à fils comprenant une pluralité d'éléments de fil (805, 805'),
dans lequel le cadre de stent à fils comprend un membre proximal (801, 801') et au moins deux membres distaux (802, 803 ; 802', 803'),
dans lequel le membre proximal (801, 801') et les au moins deux membres distaux (802, 803 ; 802', 803') convergent au niveau d'une fourche du cadre de stent à fils ;
**caractérisé en ce que** :
la pluralité d'éléments de fil (805, 805') sont tissés ensemble et forment une myriade de cellules de fil fermées avec un diamètre de cellule de moins de 0,5 mm.

2. Dispositif de déviation d'écoulement ramifié (800, 800') selon la revendication 1, dans lequel la pluralité d'éléments de fil (805, 805') forment un stent à fils auto-expansible en zigzag.

3. Dispositif de déviation d'écoulement ramifié (800, 800') selon la revendication 1 ou 2, dans lequel un diamètre de la pluralité d'éléments de fil (805, 805') est inférieur ou égal à 0,0127 cm, de préférence 0,00254 cm.

4. Dispositif de déviation d'écoulement ramifié (800, 800') selon l'une des revendications 1 à 3, dans lequel la pluralité d'éléments de fil (805, 805') pour chaque membre (801, 802, 803 ; 801', 802', 803') du cadre de stent à fils comprend 48-60 fils.

5. Dispositif de déviation d'écoulement ramifié (800, 800') selon l'une des revendications 1 à 4, dans lequel le cadre de stent à fils a une configuration en forme de Y.

6. Dispositif de déviation d'écoulement ramifié (800, 800') selon l'une des revendications 1 à 5, dans lequel chaque membre comprend une forme tubulaire et une lumière.

7. Dispositif de déviation d'écoulement ramifié (800, 800') selon l'une des revendications 1 à 6, dans lequel le cadre de stent à fils est fabriqué à partir de Nitinol.

8. Dispositif de déviation d'écoulement ramifié (800, 800') selon l'une des revendications 1 à 7, comprenant en outre une couche de base fixée au cadre de stent à fils et entourant au moins l'une de ses parties, la couche de base étant formée avec un matériau de greffon poreux ayant une pluralité de fentes formées dans une direction longitudinale du dispositif de déviation d'écoulement (800, 800'), dans lequel les fentes ont une forme différente dans une configuration comprimée et dans une configuration expansée.

9. Dispositif de déviation d'écoulement ramifié (800, 800') selon la revendication 8, dans lequel, dans la configuration comprimée, les fentes sont droites et dans la configuration expansée, les fentes ont une forme de diamant ou une forme rhomboïde.
